# EUROPEAN PATENT SPECIFICATION

(11) **EP 0 262 785 B1**
(45) Date of publication and mention of the grant of the patent: **20.10.1993**
(21) Application number: 87307446.2
(22) Date of filing: 24.08.1987
(51) Int. Cl.: C07C 217/54, A61K 31/135

(54) **N-[2-(4-carboxymethoxyphenyl)-1-methylethyl]-2-hydroxy-2-(3-chlorophenyl)ethane amine, the methyl ester, salt and stereoisomeric forms thereof**
N-[2-(4-Carboxymethoxyphenyl)-1-methylethyl]-2-hydroxy-2-(3-chlorophenyl)-ethanamine, ihre Methylester, Salze oder Stereoisomere
N-[2-(4-carboxyméthoxyphényl)-1-méthyléthyl]-2-hydroxy-2-(3-chlorophényl)-éthanamine, son ester méthylique, ses sels et formes stéréoisomères

(30) Priority: 29.08.1986 GB 8620951
(43) Date of publication of application: 06.04.1988
(73) Proprietor: BEECHAM GROUP PLC, Brentford, Middlesex TW8 9BD (GB)
(72) Inventor: Ainsworth, Anthony Trevor, The Pinnacles Harlow Essex CM19 5AD (GB); Smith, David Glynn, Epsom Surrey KT18 5XQ (GB); Arch Jonathan Robert Sanders, Epsom Surrey KT18 5XQ (GB)
(74) Representative: Russell, Brian John

(56) References cited:
- EP-A- 0 023 385
- EP-A- 0 070 134
- EP-A- 0 170 121

## Description

This invention relates to one stereoisomeric form of certain phenylethanolamine derivatives, to a process for preparing such isomers, to pharmaceutical compositions containing them and to the use of such isomers and compositions in medicine and agriculture.

European patent Number 0,023,385 discloses compounds of the general formula (A): 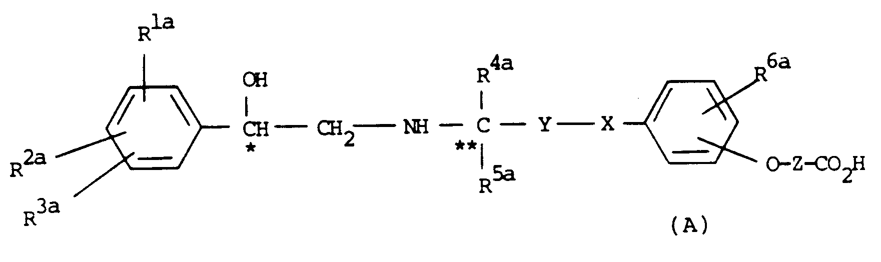
or a pharmaceutically acceptable salt, ester or amide thereof
wherein R^{1a} is a hydrogen, fluorine or chlorine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group, R^{2a} is a hydrogen, fluorine or chlorine atom or a hydroxyl group; R^{3a} is a hydrogen or chlorine atom or a hydroxyl group; or each independently represent a bromine atom; R^{4a} is a hydrogen atom or a methyl group; R^{5a} is a hydrogen atom or a methyl group; R^{6a} is a hydrogen, fluorine or chlorine atom or a methyl, methoxyl or hydroxy group; X is an oxygen atom or a bond; Y is an alkylene group of up to 6 carbon atoms or a bond; and Z is an alkylene, alkenylene or alkynylene group of up to 10 carbon atoms.

The compounds of formula (A) are disclosed as having anti-obesity and/or anti-hyperglycaemic properties.

EP 0,023,385 further discloses that the compounds of formula (A) have two asymmetric centres (when R^{4a} is different from R^{5a}), one at the carbon atom marked with a single asterisk and the other at the carbon atom marked with two asterisks. The compounds of formula (A) may therefore exist in four stereoisomeric forms.

One compound specifically disclosed in EP 0,023,385 is N-[2-(4-carbomethoxymethoxyphenyl)-1-methylethyl]-2-hydroxy-2-(3-chlorophenyl)ethanamine as a 58:42 mixture of diastereoisomers. The RR/SS racemate of this compound has also been disclosed as an intermediate in European Patent Publication No. 0,164,700.

It has now been discovered that a particular stereoisomeric form of a particular compound falling within the scope of formula (A) is especially useful as an anti-hyperglycaemic and/or anti-obesity agent.

Accordingly, the present invention provides N-[2-(4-carboxymethoxyphenyl)-1-(R)-1-methylethyl]-2-(R)-2-hydroxy-2-(3-chlorophenyl)ethanamine or a pharmaceutically acceptable salt thereof, ('the RR-isomer') optionally in admixture with N-[2-(4-carboxymethoxyphenyl)-1-(S)-1-methylethyl]-2-(S)-2-hydroxy-2-(3-chlorophenyl)ethanamine or a pharmaceutically acceptable salt thereof, ('the SS-isomer') wherein the SS-isomer forms less than 50% by weight of the mixture of the RR- and SS- isomers.

Suitably, the SS-isomer forms 0 to 45%, 0 to 40% or 0 to 30% by weight of the mixture.

Favourably, the SS- isomer forms 0 to 20% by weight of the mixture.

More favourably, the SS- isomer forms 0 to 10% by weight of the mixture.

Preferably the SS- isomer forms 0 to 5% by weight of the mixture.

More preferably, the SS- isomer forms 0 to 2% by weight of the mixture.

Most preferably the RR- isomer is in admixture with as little as possible of the SS- isomer.

It will be appreciated that the present invention encompasses the RR- isomer, in admixture as defined above with the SS- isomer and optionally in admixture with any other stereoisomer of N-[2-(4-carboxymethoxyphenyl)-1-methylethyl]-2-hydroxy-2-(3-chlorophenyl)ethanamine or a pharmaceutically acceptable salt thereof, that is with N-[2-(4-carboxymethoxyphenyl-1-(R)-1-methylethyl]-2-(S)-2-hydroxy-2-(3-chlorophenyl)ethanamine or a pharmaceutically acceptable salt thereof, ('the SR- isomer') and/or N-[2-(4-carboxy-methoxyphenyl)-1-(S)-1-methylethyl]-2-(R)-2-hydroxy-2-(3-chlorophenyl)ethanamine or a pharmaceutically acceptable salt thereof, ('the RS- isomer').

As stated above however a preferred aspect of the present invention provides the RR- isomer in admixture with as little as possible of the SS- isomer. Accordingly in one particular aspect of the invention there is provided the RR- isomer, admixed with as little as possible of the SS- isomer, and optionally in admixture with either the RS- isomer or the SR- isomer.

Suitably, the RR- isomer, admixed with as little as possible of the SS- isomer, is optionally in admixture with the RS- isomer wherein the RS- isomer forms up to 50% by weight of the mixture .

Suitably, the RR- isomer, admixed with as little as possible of the SS- isomer, is optionally in admixture with the SR- isomer wherein the SR- isomer forms up to 50% by weight of the mixture .

Suitably, the RS- or SR- isomer forms 0 to 45%, 0 to 40%, 0 to 30%, 0 to 20%, 0 to 10%, 0 to 5% or especially 0 to 2% of the mixture.

Most preferably the RR isomer is in admixture with as little as possible of the RS- isomer or the SR- isomer.

It will be appreciated from the foregoing that an especially preferred aspect of the present invention is that wherein the RR- isomer is in admixture with as little as possible of the SS- isomer or the RS- isomer or the SR- isomer.

In its most preferred aspect the present invention therefore provides the RR- isomer in optically pure form.

When used herein, unless specifically indicated to the contrary, all percentages (%) relate to percentages by weight.

Suitable pharmaceutically acceptable salts include acid addition salts and carboxyl group salts.

Suitable pharmaceutically acceptable salts include acid addition salts, in particular the compounds of this invention which are esterified may be provided as acid addition salts. Such salts may be of an organic or inorganic acid but are normally salts with a pharmaceutically acceptable acid. Suitable acid addition salts include those formed with acids such as hydrochloric, hydrobromic, orthophosphoric, sulphuric, methanesulphonic, toluenesulphonic, acetic, propionic, lactic, citric, fumaric, malic, succinic, salicylic, acetylsalicylic or the like acid.

Preferred acid addition salts are the hydrochloride, hydrobromide, fumarate and hemi-fumarate, especially the hemi-fumarate.

Suitable pharmaceutically acceptable carboxyl group salts include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β- phenethylamine, dehydroabietylamine, N,N'-bisdehydroabietylamine, glucamine, N-methylglucamine or bases of the pyridine type such as pyridine, collidine or guinoline.

A preferred carboxyl group salt is the sodium salt.

The present invention also provides a process for preparing the RR- isomer in admixture with the SS-isomer wherein the SS- isomer forms less than 50% by weight of the mixture of the RR- and SS- isomers, which process comprises resolution of a racemic mixture of the RR- isomer and the SS- isomer.

The term 'resolution' as used herein relates to the separation of a racemic mixture of enantiomers into two fractions one of which is enriched in one enantiomer relative to the original mixture.

Resolution of the racemic mixture of the RR- isomer and SS- isomer may be effected by any convenient means.

Accordingly, there is provided a process for preparing the RR- isomer which process comprises:
1) derivatising the mixture with a chiral derivatising agent, to form a mixture of diastereoisomers;
2) separating the mixture of diastereoisomers, for example by fractional crystallization, to give a diastereoisomer formed by the RR- isomer optionally in admixture with a diastereoisomer formed by the SS- isomer, wherein the diastereoisomer formed by the SS- isomer forms less than 50% by weight of the mixture of the diastereoisomer formed by the RR- isomer and the diastereoisomer formed by the SS- isomer (the required fraction), from the residue;
3) converting the required fraction from step 2) into the RR- isomer optionally in admixture with the SS- isomer, wherein the SS- isomer forms less than 50% by weight of the mixture of the RR- and SS- isomers;
4) recycling the residue via steps 1), 2) and 3).

A suitable chiral derivatising agent is an optically active acid such as those described in Topics in Stereochemistry, Vol. 6, Wiley Interscience 1971, Allinger N.L. and Eliel W.D. (Eds); suitable resolution conditions are those used conventionally in the art such as those disclosed in the abovementioned Topics in Stereochemistry.

It will be appreciated that the separation of the mixture of diastereoisomers in step 2) above includes a separation wherein the required fraction is in crystalline form and the residue is the mother liquor or conversely that the required fraction is the mother liquor and the residue is in crystalline form.

Preferably the required fraction is in crystalline form.

The racemic mixture of any RR- isomer and the SS- isomer may be prepared for example by using methods disclosed in EP 0,023,385.

As stated above a most preferred aspect of the present invention provides the RR- isomer admixed with as little as possible of the SS- isomer. A suitable means of attaining this most preferred aspect is by way of a stereoselective synthesis.

Accordingly in a most preferred aspect the present invention also provides a process for preparing N-[2-(4-carboxymethoxyphenyl)-1-(R)-1-methylethyl]-2-(R)-2-hydroxy-2-(3-chlorophenyl)-ethanamine or a pharmaceutically acceptable salt thereof, admixed with as little as possible of the SS- isomer, and optionally in admixture with the RS- isomer or the SR- isomer wherein the RS- isomer forms up to 50% by weight of the mixture between the RR- and RS- isomers or the SR- isomer forms up to 50% by weight of the mixture between the RR- and SR- isomers, which process comprises reacting a compound of formula (IA) with a compound of formula (IB): 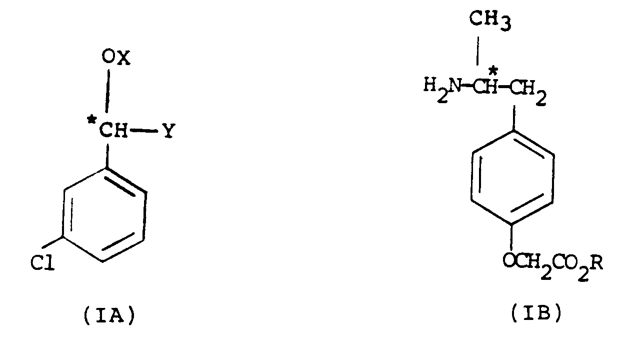
wherein X represents hydrogen and Y represents -CH₂R^{x} wherein R^{x} is a leaving group or X together with Y represents a moiety 〉CH₂ and R represents a hydrogen atom or a protecting group and either, one of the asterisked carbon atoms has the R- configuration and the other asterisked carbon is a racemic carbon or both asterisked carbon atoms have the R- configuration; and thereafter if required carrying out one or more of the following optional steps:
(i) separating any mixture of epimers;
(ii) removing any protecting group;
(iii) forming the methyl ester of any group -OCH₂CO₂H;
(iv) forming a pharmaceutically acceptable salt of the product so formed.

Suitably, both of the asterisked carbons have the R-configuration.

A suitable protecting group, R, is a C₁₋₆ alkyl group.

A suitable leaving group R^{x} is a halogen atom, preferably a bromine atom, a mesyloxy group or a tosyloxy group, preferably a tosyloxy group.

The reaction between compounds of formulae (IA) and (IB) may be carried out in any suitable solvent at low to elevated temperatures. For example for compounds wherein X is hydrogen and Y is a moiety -CH₂R^{x} the reaction is conveniently carried out in dimethylsulphoxide at a temperature of from 30 to 80°C, advantageously at about 50°C, over a period of 1 to 4 days, such as 3 days; or for compounds wherein X together with Y represents 〉CH₂ the reaction is conveniently carried out in a protic solvent such as a lower alkanol having at least two carbon atoms, preferably ethanol, suitably at the reflux temperature of the solvent.

Step (i) may be carried out conventionally by for example fractional crystallization of the mixture of epimers.

The compounds of formula (IA) and (IB) are known compounds or may be prepared using methods analogous to those used to prepare known compounds.

The present invention also provides a process for preparing the RR- isomer, in admixture with as little as possible of the SS- isomer, optionally in admixture the RS- isomer or the SR- isomer, wherein the RS-isomer forms up to 50% by weight of the mixture between the RR- and RS- isomers or the SR- isomer forms up to 50% by weight of the mixture between the RR- and SR- isomers which process comprises reducing a compound of formula (II): 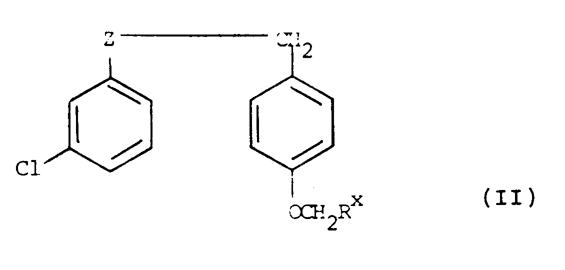
wherein R^{x} is a carboxyl group or a protected form thereof and Z represents a moiety of formula (c), (d), (e) or (f): 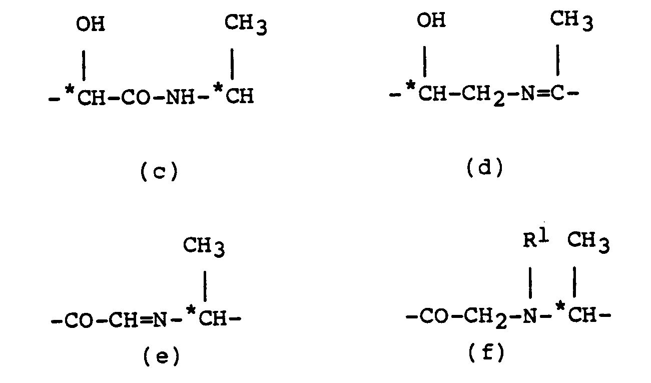
and in compounds of formula (II) wherein Z is a moiety (c), either one of the asterisked carbon atoms has the R- configuration and the other asterisked carbon is a racemic carbon or both asterisked carbons have the R- configuation; and in compounds wherein Z is a moiety (d), (e) or (f) the asterisked carbon has the R- configuration; R¹ in the moiety (f) represents hydrogen or a protecting group, such as a benzyl group; and thereafter if required carrying out one or more of the following optional steps:
(i) separating any mixture of epimers;
(ii) removing any protecting group;
(iii) forming a carboxyl group from any protected carboxyl group;
(iv) forming a pharmaceutically acceptable of the product so formed.

Suitably, the reduction of the compound of formula (II), wherein Z is a moiety (c), (e) or (f), may be carried out by using a complex metal hydride, such as sodium borohydride, or diborane; the reduction being generally carried out in any suitable solvent (for example the sodium borohydride reduction may be carried out in a C₁₋₆ alkanolic solvent, such as methanol, and the diborane reduction may be carried out in tetrahydrofuran) suitably at ambient temperature for example at 20°C to 30°C.

Normally, the reduction of the compound of formula (II), wherein Z is a moiety (d), may be carried out by catalytic hydrogenation. Suitably catalysts include noble metal catalysts such as palladium, for example palladium on such as charcoal, or platinum as for example platinum oxide.

A suitable protected form of the carboxyl group for the complex metal hydride reduction of a compound of formula (II) includes a salted carboxyl group, preferably wherein the salting ion is a metal ion such as a sodium ion.

A suitable protected form of the carboxyl group for the catalytic hydrogenation of a compound of formula (II) is a C₁₋₆ alkyl ester, preferably a methyl ester.

Preferably, in the compound of formula (II) wherein Z is a moiety (c) both asterisked carbons have the R- configuration.

The compounds of the formula (II), wherein Z is a moiety (c), may be prepared by reaction between compounds of formulae (IIIA) and (IIIB): 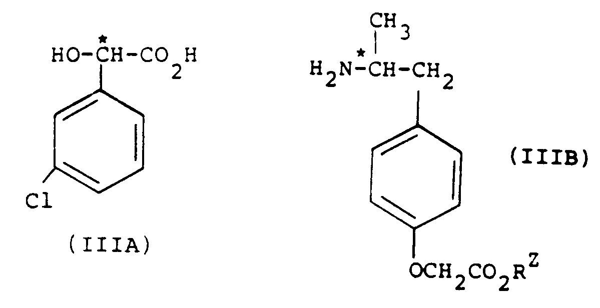
wherein R^{z} represents a protecting group, such as a methyl or benzyl group, and either one of the asterisked carbons has the R- configuration and the other asterisked carbon is a racemic carbon or both asterisked carbons have the R- configuration; and thereafter converting the moiety -CO₂R^{z} to a group R^{x} as defined in relation to formula (II).

Suitable reaction conditions are standard peptide formation conditions, for example by reaction in the presence of dicyclohexylcarbodi-imide, 1-hydroxybenztriazole and dimethylformamide.

Preferably, both asterisked carbons have the R- configuration.

The compounds of formulae (IIIA) and (IIIB) are known compounds or may be prepared by methods analogous to those used to prepare known compounds.

The compounds of formula (II), wherein Z is a moiety (d), may be prepared by reaction between compounds of formulae (IVA) and (IVB): 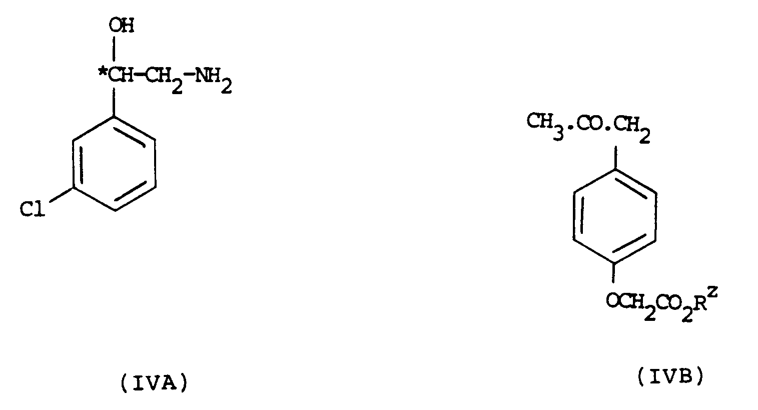
wherein R^{z} is as defined in relation to formula (IIIB) and the asterisked carbon in (IVA) has the R- configuration; and thereafter if required converting the moiety -CO₂R^{z} to a group R^{x} as defined in relation to formula (II).

Suitable reaction conditions are those which involve removal of water formed in the reaction, for example those which involve azeotropic removal of water from any suitable refluxing reaction solvent, such as benzene, using a Dean and Stark apparatus.

In a preferred form of the reaction the compound of formula (II), wherein Z is a moiety (d), is not isolated from the reaction between the compounds of formulae (IVA) and (IVB), it is reduced in situ to a compound of the invention.

The compounds of formula (IVA) and (IVB) are either known compounds or they may be prepared by methods analogous to those used for known compounds.

The compounds of formula (II), wherein Z is a moiety (e) may be prepared by reaction between (3-chlorophenyl)glyoxal and a compound of the hereinbefore defined formula (IIIB); and thereafter converting the moiety -CO₂R^{z} to a group R^{x} as defined in relation to formula (II).

Suitably the preceding reaction is carried out under the same conditions as for the reaction between the compounds of formulae (IVA) and (IVB)
The compounds of formula (II), wherein Z is a moiety (f), may be prepared by reaction between compounds of formulae (VA) and (VB): 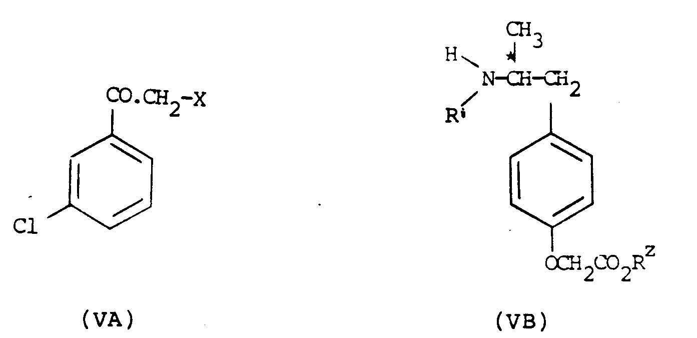
wherein X represents a halogen atom, preferably a bromine atom, R^{z} is as defined in relation to formula (IIIB), the asterisked carbon in formula (VB) has the R- configuration and R¹ is as defined in relation to moiety (f) of formula (II); and thereafter converting a moiety -CO₂R^{z} to a group R^{x} as defined in relation to formula (II).

The reaction between the compounds of formula (VA) and (VB) may be carried out in a solvent such as acetonitrile or butanone at an elevated temperature for example at the reflux temperature of the reaction solvent, and normally in the presence of an acid acceptor for example a further mole of a compound of formula (VB).

The compounds of formulae (VA) and (VB) are either known compounds or may be prepared using methods analogous to those used to prepare known compounds.

The moiety -CO₂R^{z} may be converted to the group R^{x} by any convenient method, for example when R^{z} is methyl the moiety CO₂CH₃ may be hydrolysed under basic conditions using sodium hydroxide to yield R^{z} as a sodium salted carboxyl group.

The absolute configuration of each stereoisomer of the invention may be determined using conventional X-ray crystallographic techniques.

As previously indicated, the compounds of the present invention have valuable pharmacological properties.

The present invention also provides the RR- isomer optionally in admixture with the SS- isomer, wherein the SS- isomer forms less than 50% by weight of the mixture between the RR- and SS- isomers, for use as an active therapeutic substance.

In one aspect, the present invention provides the RR- isomer optionally in admixture with the SS- isomer, wherein the SS- isomer forms less than 50% by weight of the mixture between the RR- and SS- isomers, for use in the treatment of hyperglycaemia in human or non-human mammals.

The present invention further provides the RR- isomer optionally in admixture with the SS- isomer, wherein the SS- isomer forms less than 50% by weight of the mixture between the RR- and SS- isomers, for use in the treatment of obesity in human or non-human mammals.

The compound of the invention may be administered per se or, preferably, as a pharmaceutical composition also comprising a pharmaceutically acceptable carrier.

Accordingly, the present invention also provides a pharmaceutical composition, comprising the RR- isomer optionally in admixture with the SS- isomer, wherein the SS- isomer forms less than 50% by weight of the mixture between the RR- and SS- isomers, the composition also comprising a pharmaceutically acceptable carrier.

As used herein the term 'pharmaceutically acceptable' embraces compounds, compositions and ingredients for both human and veterinary use: for example the term 'pharmaceutically acceptable salt' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection, are also envisaged.

In accordance with conventional pharmaceutical practice the carrier may comprise a diluent, filler, disintegrant, wetting agent, lubricant, colourant, flavourant or other conventional adjuvant.

Particularly suitable compositions for oral administration are unit dosage forms such as tablets and capsules. Other fixed unit dosage forms, such as powders presented in sachets, may also be used.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose form will normally contain an amount of the active ingredient in the range of from 0.01 to 1000 mg, usually 0.1 to 500 mg, more usually 0.1 to 250 mg, especially 0.1 to 100mg and more especially 0.1 to 50 mg. Typical unit dose compositions will comprise 0.1 to 25 mg, 0.1 to 15 mg, 1 to 15 mg or 1 to 10 mg of the active ingredient.

Conveniently, the active ingredient may be administered as a pharmaceutical composition hereinbefore defined, and this forms a particular aspect of the present invention.

In treating hyperglycaemic or obese humans the compounds of the invention thereof may be taken in doses, such as those described above, one to six times,preferably one to four times, a day in a manner such that the total daily dose for a 70 kg adult will generally be in the range of from 0.1 to 6000 mg, usually about 1 to 3000 mg, more usually about 1 to 1500 mg especially 1 to 600 mg and more especially 1 to 300 mg. In general a total daily dose of from about 1 x 10⁻³ mg/kg to 100 mg/kg, usually from about 0.1 mg/kg to 50 mg/kg, more usually from about 0.1 mg/kg to 25 mg/kg, especially from about 0.1 mg/kg to 10 mg/kg and more especially from about 0.1 mg/kg to 5 mg/kg may be used.

In treating hyperglycaemic or obese non-human mammals, especially dogs, the active ingredient may be adminstered by mouth, usually once or twice a day and in an amount in the range of from about 0.1 µg/kg to 1 mg/kg, for example 10 µg/kg to 0.1 mg/kg.

In a further suitable aspect the present invention also provides a method for improving the weight gain and/or improving the feed utilisation efficiency and/or increasing lean body mass; of livestock, which method comprises the administration to livestock of an effective non-toxic amount of the RR- isomer optionally in admixture with the SS- isomer, wherein the SS-isomer forms less than 50% by weight of the mixture between the RR- and SS- isomers.

Suitably, this aspect of the invention provides the RR- isomer optionally in admixture with the SS- isomer, wherein the SS- isomer forms less than 50% by weight of the mixture between the RR- and SS- isomers, for use in decreasing birth mortality rate and increasing the post-natal survival rate of livestock.

Suitable livestock include poultry (especially turkeys and chickens), cattle, pigs, sheep or goats.

Suitably, this aspect of the invention provides a method for increasing the weight gain and/or improving the feed utilisation efficiency and/or increasing the lean body mass of poultry, (especially turkeys and chickens), cattle, pigs or sheep; particularly of cattle, pigs or sheep.

It will be understood that the advantages provided by this aspect of the present invention relating to the decrease in birth mortality rate and increase in post-natal survival rate are provided by administration to female parent livestock or the newly-born livestock, the decrease in birth mortality rate and increase in post-natal survival rate relating primarily to the newly born livestock.

Suitable pregnant livestock include pregnant cows, sows and ewes.

The method of the invention is particularly suitable for decreasing the birth mortality rate and increasing the post-natal survival rate of lambs by administration to pregnant ewes.

In the preceding methods the compounds of the invention will normally be administered orally although non-oral modes of administration, for example injection or implantation, are also envisaged. Suitably the compounds are administered in the feed-stuff or drinking water provided for the livestock. Conveniently these are administered in the feed-stuff at from 10⁻³ ppm - 500ppm of total daily feed intake, more usually 0.01ppm to 250ppm, suitably less than 100ppm.

The particular formulations used will of course depend upon the mode of administration but will be those used conventionally in the mode of administration chosen.

For administration in feed-stuff the compounds of the invention are conveniently formulated as a premix in association with a suitable carrier.

Accordingly, the present invention also provides a veterinarily acceptable premix formulation comprising the RR- isomer optionally in admixture with the SS- isomer, wherein the SS- isomer forms less than 50% by weight of the mixture between the RR- and SS- isomers, the premix formulation also comprising a veterinarily acceptable carrier.

Suitable carriers are inert conventional agents such as powdered starch. Other conventional feed-stuff premix carriers may also be employed.

No toxicological effects are indicated when a compound of the invention is administered in any of the abovementioned dosage ranges.

### Procedure

### N-[2-(4-Carbomethoxymethoxy phenyl)-1-(R)-1-methyl ethyl-2-(R)-2-hydroxy-2-(3-chlorophenyl)ethananine, hemifumarate

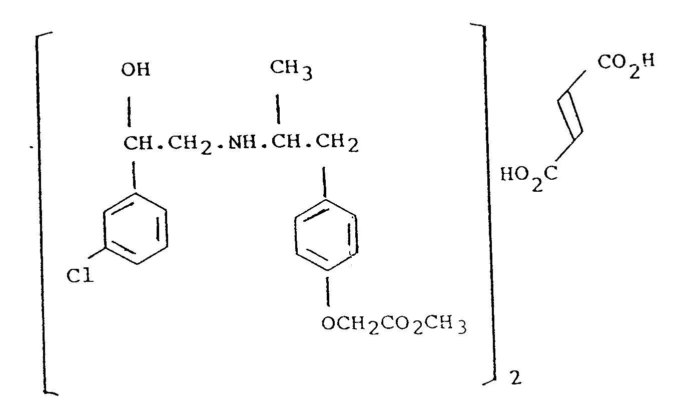

A mixture of (R)-3-chlorophenyl oxirane (O.9g.) and (R)-methyl 4-[2-aminopropyl]phenoxy acetate (1.3g.) in dry dimethylsulphoxide (80ml) was stirred and heated at 70°C for 3 days. The cooled reaction mixture was poured into water and extracted into ethyl acetate. The organic phase was washed with water, dried (MgSO₄) and evaporated to an oil which was purified by column chromatography on silica gel. Elution with 4% methanol in chloroform gave N-[2-(4-carbomethoxymethoxyphenyl)-1(R)-1-methylethyl]-2-(R)-2-hydroxy-2-(3-chlorophenyl) ethanamine which was isolated as the hemi-fumarate salt, m.pt. 80-83°(ethylacetate), [α]D²⁵(MeOH)-33.4°.

### 'H nmr

δ(DMSO)-d₆): 0.96(3H,s); 2.32-3.20(5H,m); 3.70(3H,s); 4.68-4.85(2H,s plus 1H,m); 6.50(1H,s); 6.65-7.43(11H,m 3H replaceable by D₂O).

### DEMONSTRATION OF EFFECTIVENESS OF COMPOUND

### (a) Anti-hyperglycaemic activity

Female CFLP mice, weighing approximately 25 g, were fasted for 24 hours prior to the study. The compound under study was administered orally as an aqueous solution to each of 6 mice. 30 minutes later a blood sample (10µl) was obtained from the tail for the analysis of blood glucose. Immediately after taking this blood sample, glucose (1g/kg body weight) was administered subcutaneously to each mouse. 6 mice were given water as a control. Blood samples were then obtained from each mouse at 30 minute intervals for 120 minutes.

Compounds that produce a significant (P<0.05) reduction of blood glucose, compared with control mice given water, at any time interval, are considered active. The area under the blood glucose curve over the 2 hour period after the administration of the glucose was calculated for the compound and compared with the value for control animals. The result for the compound is as follows:

| Dose (µmol/kg) | % Reduction in area under Blood Glucose Curve |
|---|---|
| 0.02 | 51 |

### (b) Effect on Energy Expenditure of Rats

The effect of the compounds on the energy expenditure of rats was demonstrated by means of the following procedure:
Male Sprague-Dawley rats each weighing between 170-200 g were deprived of food for 16 hours before, and during the experiment. Water was provided ad lib at all times. The compounds were administered orally in water to 3 or 4 rats. A further 4 rats were dosed orally with water. The rats were placed in boxes through which air was drawn and the oxygen content of the air leaving the boxes was measured. The energy expenditure of the rats was calculated for 3 hours and for 21 hours after dosing from the volume of air leaving the boxes and its oxygen content, following the principles described by J.B. de V. Weir, J. Physiol. (London) 109, 1-9 (1949). The results are expressed as a percentage of the rate of energy expenditure of the rats dosed with water.

| Dose (µmol/kg p.o.) | Mean Energy Expenditure | |
|---|---|---|
| | (0-3h) | (0-21h) |
| 0.2 | 134 | 111 |

## Claims (Claims for the following Contracting State(s): BE, CH, DE, FR, GB, GR, IT, LI, LU, NL, SE)

1. A compound, N-[2-(4-carboxymethoxyphenyl)-1-(R)-1-methylethyl]-2-(R)-2-hydroxy-2-(3-chlorophenyl) ethanamine or a pharmaceutically acceptable salt thereof, ('the RR-isomer') optionally in admixture with N-[2-(4-carboxymethoxyphenyl)-1-(S)-1-methylethyl]-2-(S)-2-hydroxy-2-(3-chlorophenyl)ethanamine or a pharmaceutically acceptable salt thereof ('the SS-isomer'), characterised in that the SS-isomer forms less than 50% by weight of the mixture of the RR- and SS- isomers.

2. A compound according to claim 1, wherein the SS-isomer forms 0 to 45%, 0 to 40% or 0 to 30% by weight of the mixture.

3. A compound according to claim 1 or claim 2, wherein the SS-isomer forms 0 to 10% by weight of the mixture.

4. A compound according to any one of claims 1 to 3, wherein the SS-isomer forms 0 to 2% by weight of the mixture.

5. A compound according to any one of claims 1 to 4, wherein the RR-isomer is in admixture with as little as possible of the SS-isomer.

6. A compound according to any one of claims 1 to 5, wherein the RR-isomer is in optically pure form.

7. A process for preparing N-[2-(4-carboxymethoxyphenyl)-1-(R)-1-methylethyl]-2-(R)-2-hydroxy-2-(3-chlorophenyl)ethanamine or a pharmaceutically acceptable salt thereof, ('the RR-isomer') optionally in admixture with N-[2-(4-carboxymethoxyphenyl)-1-(S)-1-methylethyl]-2-(S)-2-hydroxy-2-(3-chlorophenyl)ethanamine, or a pharmaceutically acceptable salt thereof, ('the SS-isomer') wherein the SS-isomer forms less than 50% by weight of the mixture of the RR- and SS-isomers; which process comprises,
resolution of a racemic mixture of the RR-isomer and the SS-isomer; or
a process for preparing N-[2-(4-carboxymethoxy phenyl)-1-(R)-1-methylethyl]-2-(R)-2-hydroxy-2- (3-chlorophenyl)-ethanamine or a pharmaceutically acceptable salt thereof, admixed with as little as possible of the SS-isomer, and optionally in admixture with the RS-isomer or the SR-isomer wherein the RS-isomer forms up to 50% by weight of the mixture between the RR- and RS-isomers or the SR-isomer forms up to 50% by weight of the mixture between the RR- and SR-isomers, characterised in that the process comprises:
(A) reacting a compound of formula (IA) with a compound of formula (IB): 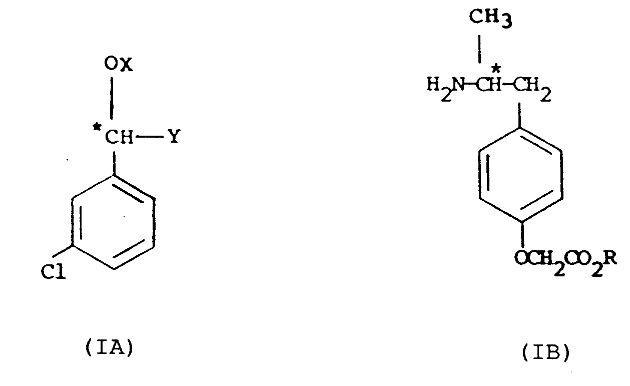 wherein X represents hydrogen and Y represents -CH₂R^{x} wherein R^{x} is a leaving group or X together with Y represents a moiety 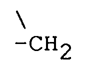 and R represents a hydrogen atom or a protecting group and either, one of the asterisked carbon atoms has the R-configuration and the other asterisked carbon is a racemic carbon or both asterisked carbon atoms have the R-configuration; or
(B) reducing a compound of formula (II): 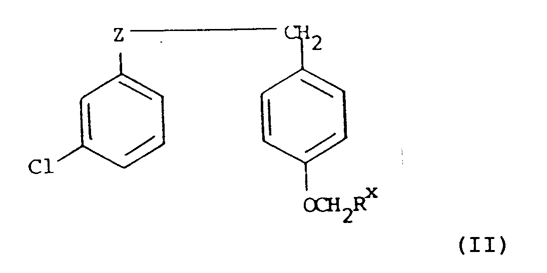 wherein R^{x} is a carboxyl group or a protected form thereof and Z represents a moiety of formula (c), (d), (e) or (f): 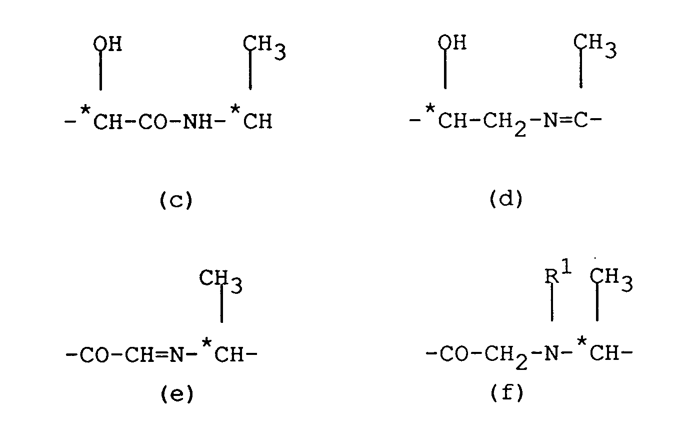 and in compounds of formula (II) wherein Z is a moiety (c), either one of the asterisked carbon atoms has the R-configuration and the other asterisked carbon is a racemic carbon or both asterisked carbons have the R-configuation; and in compounds wherein Z is a moiety (d), (e) or (f) the asterisked carbon has the R-configuration; R¹ in the moiety (f) represents hydrogen or a protecting group;
and thereafter if required carrying out one or more of the following optional steps:
(i) separating any mixture of epimers;
(ii) removing any protecting group;
(iii) forming a carboxyl group from any protected carboxyl group;
(iv) forming a methyl ester of any group -OCH₂CO₂H;
(v) forming a pharmaceutically acceptable of the product so formed.

8. The RR-isomer, as defined in claim 1, optionally in admixture with the SS-isomer, as defined in claim 1, wherein the SS-isomer forms less than 50% by weight of the mixture between the RR- and SS-isomers, for use as an active therapeutic substance.

9. The RR-isomer, as defined in claim 1, optionally in admixture with the SS-isomer, as defined in claim 1, wherein the SS-isomer forms less than 50% by weight of the mixture between the RR- and SS-isomers, for use in the treatment of hyperglycaemia in human or non-human mammals.

10. The RR-isomer, as defined in claim 1, optionally in admixture with the SS-isomer, as defined in claim 1, wherein the SS-isomer forms less than 50% by weight of the mixture between the RR- and SS-isomers, for use in the treatment of obesity in human or non-human mammals.

11. A pharmaceutical composition, comprising the RR-isomer, as defined in claim 1, optionally in admixture with the SS-isomer, as defined in claim 1, wherein the SS-isomer forms less than 50% by weight of the mixture between the RR- and SS-isomers, the composition also comprising a pharmaceutically acceptable carrier.

12. A method for improving the weight gain and/or improving the feed utilisation efficiency and/or increasing lean body mass; of livestock, which method comprises the administration to livestock of an effective non-toxic amount of the RR-isomer, as defined in claim 1, optionally in admixture with the SS-isomer, wherein the SS-isomer,as defined in claim 1, forms less than 50% by weight of the mixture between the RR- and SS-isomers.

13. The RR-isomer, as defined in claim 1, optionally in admixture with the SS-isomer, as defined in claim 1, wherein the SS-isomer forms less than 50% by weight of the mixture between the RR- and SS-isomers, for use in decreasing birth mortality rate and increasing post-natal survival rate of livestock.

14. A veterinarily acceptable premix formulation, comprising the RR-isomer, as defined in claim 1, optionally in admixture with the SS-isomer, as defined in claim 1, wherein the SS-isomer forms less than 50% by weight of the mixture between the RR- and SS-isomers, the premix formulation also comprising a veterinarily acceptable carrier therefor.

15. The RR- isomer, as defined in claim 1, optionally in admixture with the SS- isomer, as defined in claim 1, wherein the SS- isomer forms less than 50% by weight of the mixture between the RR- and SS- isomers, for use in the treatment of hyperglycaemia or obesity in human or non-human mammals.

## Claims (Claims for the following Contracting State(s): ES)

1. A process for preparing N-[2-(4-carboxymethoxyphenyl)-1-(R)-1-methylethyl]-2-(R)-2-hydroxy-2-(3-chlorophenyl)ethanamine or a pharmaceufically acceptable salt thereof, ('the RR-isomer') optionally in admixture with N-[2-(4-carboxymethoxyphenyl)-1-(S)-1-methyl-ethyl]-2- (S)-2-hydroxy-2-(3-chlorophenyl)ethanamine or a pharmaceutically acceptable salt thereof, ('the SS-isomer') wherein the SS-isomer forms less than 50% by weight of the mixture of the RR- and SS- isomers; which process comprises resolution of a racemic mixture of the RR-isomer and the SS-isomer; or
a process for preparing N-[2-(4-carboxymethoxyphenyl)-1-(R)-1-methylethyl]-2-(R)-2- hydroxy-2- (3-chlorophenyl)-ethanamine or a pharmaceutically acceptable salt thereof, admixed with as little as possible of the SS- isomer, and optionally in admixture with the RS-isomer or the SR- isomer wherein the RS- isomer forms up to 50% by weight of the mixture between the RR- and RS- isomers or the SR- isomer forms up to 50% by weight of the mixture between the RR- and SR- isomers, which process comprises:
(A) reacting a compound of formula (IA) with a compound of formula (IB): 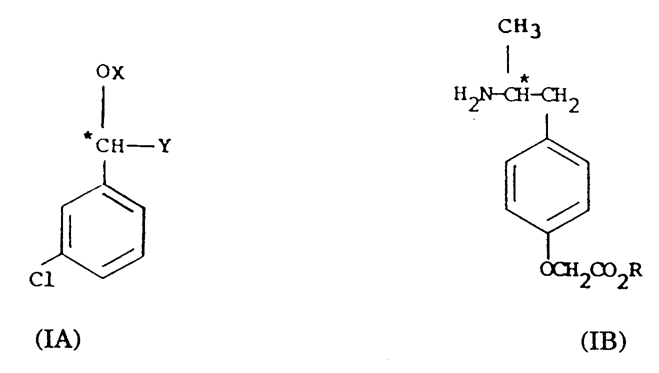 wherein X represents hydrogen and Y represents -CH₂R^{x} wherein R^{x} is a leaving group or X together with Y represents a moiety CH₂ and R represents a hydrogen atom or a protecting group and either, one of the asterisked carbon atoms has the R- configuration and the other asterisked carbon is a racemic carbon or both asterisked carbon atoms have the R- configuration; or
(B) reducing a compound of formula (II): 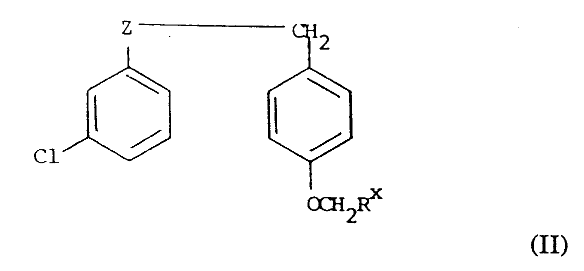 wherein R^{x} is a carboxyl group or a protected form thereof and Z represents a moiety of formula (c), (d), (e) or (f): 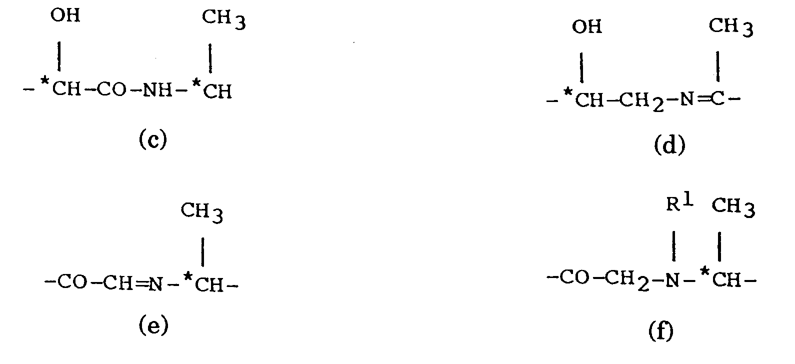 and in compounds of formula (II) wherein Z is a moiety (c), either one of the asterisked carbon atoms has the R- configuration and the other asterisked carbon is a racemic carbon or both asterisked carbons have the R- configuation; and in compounds wherein Z is a moiety (d), (e) or (f) the asterisked carbon has the R- configuration; R¹ in the moiety (f) represents hydrogen or a protecting group;
and thereafter if required carrying out one or more of the following optional steps:
(i) separating any mixture of epimers;
(ii) removing any protecting group;
(iii) forming a carboxyl group from any protected carboxyl group;
(iv) forming a pharmaceutically acceptable of the product so formed.

2. A process according to claim 1, wherein the SS-isomer forms 0 to 45%, 0 to 40% or 0 to 30% by weight of the mixture.

3. A process according to claim 1 or claim 2, wherein the SS- isomer forms 0 to 10% by weight of the mixture.

4. A process according to any one of claims 1 to 3, wherein the SS- isomer forms 0 to 2% by weight of the mixture.

5. A process according to any one of claims 1 to 4, wherein the RR-isomer is in admixture with as little as possible of the SS- isomer.

6. A process according to any one of claims 1 to 5, wherein the RR- isomer is in optically pure form.

7. The RR- isomer, as defined in claim 1, optionally in admixture with the SS- isomer, as defined in claim 1, wherein the SS- isomer forms less than 50% by weight of the mixture between the RR- and SS- isomers, for use as an active therapeutic substance.

8. The RR- isomer, as defined in claim 1, optionally in admixture with the SS- isomer, as defined in claim 1, wherein the SS- isomer forms less than 50% by weight of the mixture between the RR- and SS- isomers, for use in the treatment of hyperglycaemia in human or non-human mammals.

9. The RR- isomer, as defined in claim 1, optionally in admixture with the SS- isomer, as defined in claim 1, wherein the SS- isomer forms less than 50% by weight of the mixture between the RR- and SS- isomers, for use in the treatment of obesity in human or non-human mammals.

10. A method for improving the weight gain and/or improving the feed utilisation efficiency and/or increasing lean body mass and/or decreasing birth mortality rate and increasing post-natal survival rate; of livestock, which method comprises the administration to livestock of an effective non-toxic amount of the RR- isomer, as defined in claim 1, optionally in admixture with the SS- isomer, wherein the SS- isomer,as defined in claim 1, forms less than 50% by weight of the mixture between the RR- and SS- isomers.

11. A method for decreasing birth mortality rate and increasing post-natal survival rate of livestock, which method comprises the administration to pregnant livestock of an effective, non-toxic amount of the RR-isomer, as defined in claim 1, optionally in admixture with the SS-isomer, as defined in claim 1, wherein the SS-isomer forms less than 50% by weight of the mixture between the RR- and SS-isomers.

## Patentansprüche (Patentansprüche für folgende(n) Vertragsstaat(en): BE, CH, DE, FR, GB, GR, IT, LI, LU, NL, SE)

1. Verbindung, nämlich N-[2-(4-Carboxymethoxyphenyl)-1-(R)-1-methylethyl]-2-(R)-2-hydroxy-2-(3-chlorphenyl)ethanamin oder ein pharmazeutisch verträgliches Salz davon ("das RR-Isomer"), gegebenenfalls im Gemisch mit N-[2-(4-Carboxymethoxyphenyl)-1-(S)-1-methylethyl]-2-(S)-2-hydroxy-2-(3-chlorphenyl)ethanamin oder einem pharmazeutisch verträglichen Salz davon ("das SS-Isomer"), dadurch gekennzeichnet, daß die SS-Isomerform weniger als 50 Gew.-% des Gemisches der RR- und SS-Isomeren ausmacht.

2. Verbindung nach Anspruch 1, wobei das SS-Isomer 0 bis 45 Gew.-%, 0 bis 40 Gew.-% oder 0 bis 30 Gew.-% des Gemisches ausmacht.

3. Verbindung nach Anspruch 1 oder 2, wobei das SS-Isomer 0 bis 10 Gew.-% des Gemisches ausmacht.

4. Verbindung nach einem der Ansprüche 1 bis 3, wobei das SS-Isomer 0 bis 2 Gew.-% des Gemisches ausmacht.

5. Verbindung nach einem der Ansprüche 1 bis 4, wobei das RR-Isomer sich im Gemisch mit einer so gering wie möglichen Menge SS-Isomer befindet.

6. Verbindung nach einem der Ansprüche 1 bis 5, wobei das RR-Isomer in optisch reiner Form vorliegt.

7. Verfahren zur Herstellung von N-[2-(4-Carboxymethoxyphenyl)-1-(R)-1-methylethyl]-2-(R)-2-hydroxy-2-(3-chlorphenyl)ethanamin oder einem pharmazeutisch verträglichen Salz davon, ("das RR-Isomer"), gegebenenfalls im Gemisch mit N[2-(4-Carboxymethoxyphenyl)-1-(S)-1-methylethyl]-2-(S)-2-hydroxy-2-(3-chlorphenyl)ethanamin oder einem pharmazeutisch verträglichen Salz davon, ("das SS-Isomer"), wobei das SS-Isomer weniger als 50 Gew.-% des Gemisches der RR- und SS-Isomere ausmacht, wobei das Verfahren umfaßt
Auftrennung des razemischen Gemisches des RR-Isomers und des SS-Isomers; oder
ein Verfahren zur Herstellung von N-[2-(4-Carboxymethoxyphenyl)-1-(R)-1-methylethyl]-2-(R)-2-hydroxy-2-(3-chlorphenyl)-ethanamin oder einem pharmazeutisch verträglichen Salz davon im Gemisch mit einer so gering wie möglichen Menge SS-Isomer und gegebenenfalls mit dem RS-Isomer oder SR-Isomer, wobei das RS-Isomer bis zu 50 Gew.-% des Gemisches zwischen den RR- und RS-Isomeren ausmacht oder das SR-Isomer bis zu 50 Gew.-% des Gemisches zwischen dem RR- und SR-Isomeren ausmacht, dadurch gekennzeichnet, daß das Verfahren umfaßt:
(A) Umsetzen einer Verbindung der Formel (IA) mit einer Verbindung der Formel (IB): 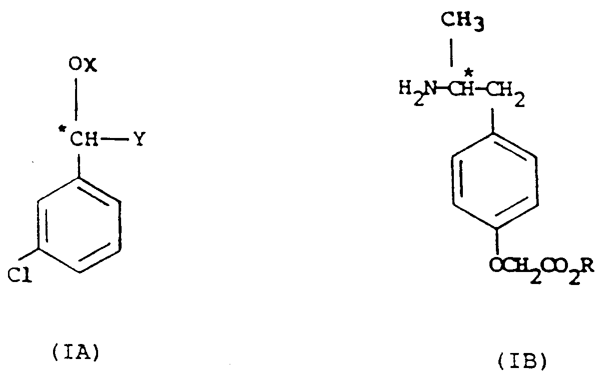 wobei X ein Wasserstoffatom darstellt und Y einen Rest der Formel -CH₂R^{x} bedeutet, wobei R^{x} eine Austrittsgruppe ist, oder X zusammen mit Y einen Rest -CH₂ darstellt und R ein Wasserstoffatom oder eine Schutzgruppe darstellt und entweder eines der mit einem Sternchen gekennzeichneten Kohlenstoffatome die R-Konfiguration aufweist und das andere mit einem Sternchen versehene Kohlenstoffatom ein razemisches Kohlenstoffatom darstellt oder beide mit einem Sternchen versehenen Kohlenstoffatome die R-Konfiguration aufweisen; oder
(B) Reduzieren einer Verbindung der Formel (II): 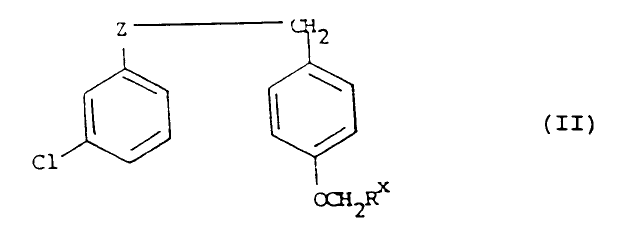 in der R^{x} eine Carboxylgruppe oder eine geschützte Form davon bedeutet und Z einen Rest der Formel (c), (d), (e) oder (f) darstellt: 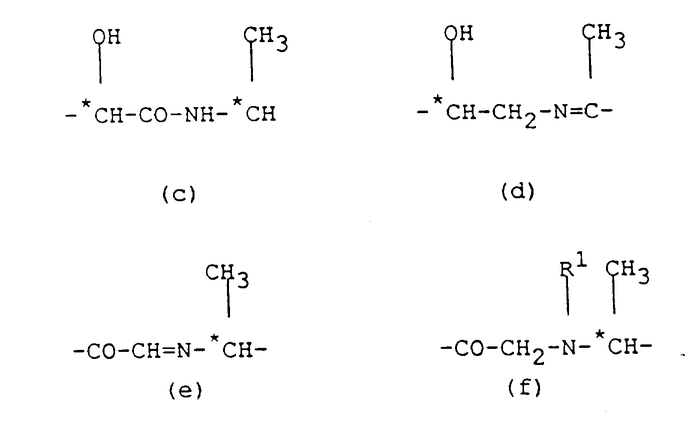 und in Verbindungen der Formel (II), in denen Z einen Rest (c) bedeutet, entweder eines der mit einem Sternchen versehenen Kohlenstoffatome die R-Konfiguration aufweist und das andere mit einem Sternchen versehene Kohlenstoffatom ein razemisches Kohlenstoffatom bedeutet oder beide mit einem Sternchen versehenen Kohlenstoffatome die R-Konfiguration aufweisen; und in Verbindungen, in denen Z einen Rest (d), (e) oder (f) darstellt, das mit einem Sternchen versehene Kohlenstoffatom die R-Konfiguration aufweist; R¹ im Rest (f) ein Wasserstoffatom oder eine Schutzgruppe darstellt;
und anschließend, falls erforderlich, Ausführen eines oder mehrerer der nachstehenden wahlweisen Schritte:
(i) Trennen eines Gemisches von Epimeren;
(ii) Entfernen von Schutzgruppen;
(iii) Herstellen einer Carboxylgruppe aus einer geschützten Carboxylgruppe;
(iv) Herstellen eines Methylesters aus Resten der Formel -OCH₂CO₂H;
(v) Herstellen eines pharmazeutisch verträglichen Salzes des so hergestellten Produkts.

8. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, zur Verwendung als therapeutischer Wirkstoff.

9. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, zur Verwendung bei der Behandlung von Hyperglykämie bei Menschen oder nicht-menschlichen Säugern.

10. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, zur Verwendung bei der Behandlung von Fettsucht bei Menschen oder nicht-menschlichen Säugern.

11. Arzneimittel, umfassend das RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomer ausmacht, wobei das Mittel auch einen pharmazeutisch verträglichen Träger umfaßt.

12. Verfahren zur Verbesserung der Gewichtszunahme und/oder Verbesserung der Futterverwertung und/oder Erhöhung der fettfreien Körpermasse von Vieh, wobei das Verfahren die Verabreichung einer wirksamen nicht-toxischen Menge des RR-Isomers gemäß Anspruch 1, gegebenenfalls im Gemisch mit dem SS-Isomer an das Vieh umfaßt, wobei das SS-Isomer gemäß Anspruch 1 weniger als 50 Gew.-% des Gemisches zwischen den RR- und SS-Isomeren ausmacht.

13. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen RR- und SS-Isomeren ausmacht, zur Verwendung bei der Verminderung der Geburtensterblichkeitsrate und der Erhöhung der post-natalen Überlebensrate von Vieh.

14. Tierarzneilich verträgliche Premix-Formulierung, umfassend das RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, und die Premixformulierung auch einen tierarzneilich verträglichen Träger dafür umfaßt.

15. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen den RR- und SS-Isomeren ausmacht, zur Verwendung bei der Behandlung von Hyperglykämie oder Fettsucht bei Menschen oder nicht-menschlichen Säugern.

## Patentansprüche (Patentansprüche für folgende(n) Vertragsstaat(en): ES)

1. Verfahren zur Herstellung von N-[2-(4-Carboxymethoxyphenyl)-1-(R)-1-methylethyl]-2-(R)-2-hydroxy-2-(3-chlorphenyl)ethanamin oder einem pharmazeutisch verträglichen Salz davon, ("das RR-Isomer"), gegebenenfalls im Gemisch mit N-[2-(4-Carboxymethoxyphenyl)-1-(S)-1-methylethyl]-2-(S)-2-hydroxy-2-(3-chlorphenyl)ethanamin oder einem pharmazeutisch verträglichen Salz davon, ("das SS-Isomer"), wobei das SS-Isomer weniger als 50 Gew.-% des Gemisches der RR- und SS-Isomere ausmacht, wobei das Verfahren umfaßt
Auftrennung des razemischen Gemisches des RR-Isomers und des SS-Isomers; oder
ein Verfahren zur Herstellung von N-[2-(4-Carboxymethoxyphenyl)-1-(R)-1-methylethyl]-2-(R)-2-hydroxy-2-(3-chlorphenyl)-ethanamin oder einem pharmazeutisch verträglichen Salz davon im Gemisch mit einer so gering wie möglichen Menge SS-Isomer und gegebenenfalls mit dem RS-Isomer oder SR-Isomer, wobei das RS-Isomer bis zu 50 Gew.-% des Gemisches zwischen dem RR- und RS-Isomeren ausmacht oder das SR-Isomer bis zu 50 Gew.-% des Gemisches zwischen dem RR- und SR-Isomeren ausmacht, dadurch gekennzeichnet, daß das Verfahren umfaßt:
(A) Umsetzen einer Verbindung der Formel (IA) mit einer Verbindung der Formel (IB): 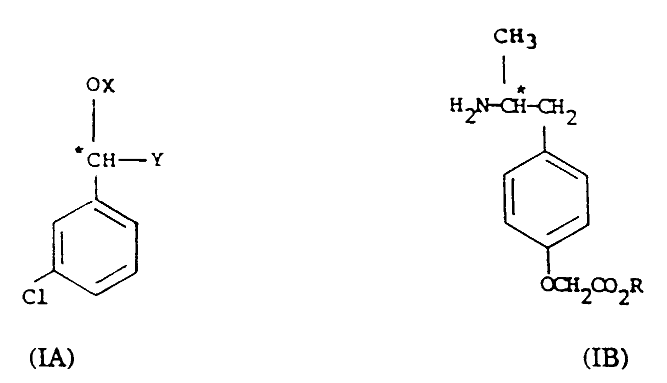 wobei X ein Wasserstoffatom darstellt und Y einen Rest der Formel -CH₂R^{x} bedeutet, wobei R^{x} eine Austrittsgruppe ist, oder X zusammen mit Y einen Rest -CH₂ darstellt und R ein Wasserstoffatom oder eine Schutzgruppe darstellt und entweder eines der mit einem Sternchen gekennzeichneten Kohlenstoffatome die R-Konfiguration aufweist und das andere mit einem Sternchen versehene Kohlenstoffatom ein razemisches Kohlenstoffatom darstellt oder beide mit einem Sternchen versehenen Kohlenstoffatome die R-Konfiguration aufweisen; oder
(B) Reduzieren einer Verbindung der Formel (II): 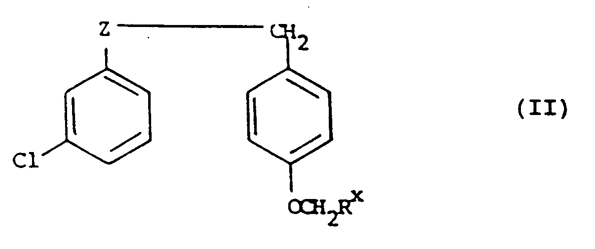 in der R^{x} eine Carboxylgruppe oder eine geschützte Form davon bedeutet und Z einen Rest der Formel (c), (d), (e) oder (f) darstellt: 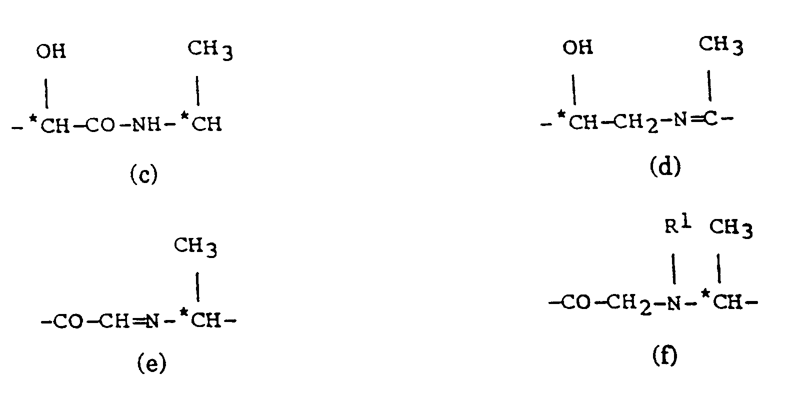 und in Verbindungen der Formel (II), in denen Z einen Rest (c) bedeutet, entweder eines der mit einem Sternchen versehenen Kohlenstoffatome die R-Konfiguration aufweist und das andere mit einem Sternchen versehene Kohlenstoffatom ein razemisches Kohlenstoffatom bedeutet oder beide mit einem Sternchen versehenen Kohlenstoffatome die R-Konfiguration aufweisen; und in Verbindungen, in denen Z einen Rest (d), (e) oder (f) darstellt, das mit einem Sternchen versehene Kohlenstoffatom die R-Konfiguration aufweist; R¹ im Rest (f) ein Wasserstoffatom oder eine Schutzgruppe darstellt;
und anschließend, falls erforderlich, Ausführen eines oder mehrerer der nachstehenden wahlweisen Schritte:
(i) Trennen eines Gemisches von Epimeren;
(ii) Entfernen von Schutzgruppen;
(iii) Herstellen einer Carboxylgruppe aus einer geschützten Carboxylgruppe;
(iv) Herstellen eines Methylesters aus Resten der Formel -OCH₂CO₂H;
(v) Herstellen eines pharmazeutisch verträglichen Salzes des so hergestellten Produkts.

2. Verfahren nach Anspruch 1, wobei das SS-Isomer 0 bis 45 Gew.-%, 0 bis 40 Gew.-% oder 0 bis 30 Gew.-% des Gemisches ausmacht.

3. Verfahren nach Anspruch 1 oder 2, wobei das SS-Isomer 0 bis 10 Gew.-% des Gemisches ausmacht.

4. Verfahren nach einem der Ansprüche 1 bis 3, wobei das SS-Isomer 0 bis 2 Gew.-% des Gemisches ausmacht.

5. Verfahren nach einem der Ansprüche 1 bis 4, wobei das RR-Isomer sich im Gemisch mit einer so gering wie möglichen Menge SS-Isomer befindet.

6. Verfahren nach einem der Ansprüche 1 bis 5, wobei das RR-Isomer in optisch reiner Form vorliegt.

7. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, zur Verwendung als therapeutischer Wirkstoff.

8. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, zur Verwendung bei der Behandlung von Hyperglykämie bei Menschen oder nicht-menschlichen Säugern.

9. RR-Isomer gemäß Anspruch 1, gegebenenfalls im Gemisch mit dem SS-Isomer gemäß Anspruch 1, wobei das SS-Isomer weniger als 50 Gew.-% des Gemisches zwischen dem RR- und SS-Isomeren ausmacht, zur Verwendung bei der Behandlung von Fettsucht bei Menschen oder nicht-menschlichen Säugern.

10. Verfahren zur Verbesserung der Gewichtszunahme und/oder Verbesserung der Futterverwertung und/oder Erhöhung der fettfreien Körpermasse von Vieh, wobei das Verfahren die Verabreichung einer wirksamen nicht-toxischen Menge des RR-Isomers gemäß Anspruch 1, gegebenenfalls im Gemisch mit dem SS-Isomer, an das Vieh umfaßt, wobei das SS-Isomer gemäß Anspruch 1 weniger als 50 Gew.-% des Gemisches zwischen dem RR- und SS-Isomeren ausmacht.

11. Verfahren zur Verminderung der Geburtensterblichkeitsrate und der Erhöhung der post-natalen Überlebensrate von Vieh, umfassend die Verabreichung einer wirksamen, nicht-toxischen Menge des RR-Isomers gemäß Anspruch 1, gegebenenfalls im Gemisch mit dem SS-Isomer gemäß Anspruch 1, wobei das SS-Isomer weniger als 50 Gew.-% des Gemisches zwischen RR- und SS-Isomeren ausmacht, an trächtige Kühe.

## Revendications (Revendications pour l'(les) Etat(s) contractant(s) suivant(s): BE, CH, DE, FR, GB, GR, IT, LI, LU, NL, SE)

1. Composé, la N-[2-(4-carboxyméthoxyphényl)-1-(R)-1-méthyléthyl]-2-(R)-2-hydroxy-2-(3-chlorophényl)-éthanamine ou un sel de celle-ci acceptable du point de vue pharmaceutique, ("l'isomère RR"), éventuellement en mélange avec la N-[2-(4-carboxyméthoxyphényl)-1-(S)-1-méthyléthyl]-2-(S)-2-hydroxy-2-(3-chloro-phényl)-éthanamine ou un sel de celle-ci acceptable du point de vue pharmaceutique, ("l'isomère SS"), caractérisé en ce que l'isomère SS forme moins de 50% en poids du mélange des isomères RR et SS.

2. Composé suivant la revendication 1, dans lequel l'isomère SS forme 0 à 45%, 0 à 40% ou 0 à 30% en poids du mélange.

3. Composé suivant les revendications 1 ou 2, dans lequel l'isomère SS forme 0 à 10% en poids du mélange.

4. Composé suivant l'une quelconque des revendications 1 à 3, dans lequel l'isomère SS forme 0 à 2% en poids du mélange.

5. Composé suivant l'une quelconque des revendications 1 à 4, dans lequel l'isomère RR est mélangé à une quantité aussi faible que possible de l'isomère SS.

6. Composé suivant l'une quelconque des revendications 1 à 5, dans lequel l'isomère RR est sous une forme optiquement pure.

7. Procédé pour préparer la N-[2-(4-carboxyméthoxyphényl)-1-(R)-1-méthyléthyl]-2-(R)-2-hydroxy-2-(3-chlorophényl)-éthanamine ou un sel de celle-ci acceptable du point de vue pharmaceutique, ("l'isomère RR"), éventuellement en mélange avec la N-[2-(4-carboxyméthoxyphényl)-1-(S)-1-méthyléthyl]-2-(S)-2-hydroxy-2-(3-chlorophényl)-éthanamine ou un sel de celle-ci acceptable du point de vue pharmaceutique, ("l'isomère SS"), où l'isomère SS forme moins de 50% en poids du mélange des isomères RR et SS, lequel procédé comprend :
la résolution d'un mélange racémique de l'isomère RR et de l'isomère SS; ou
procédé pour préparer la N-[2-(4-carboxyméthoxyphényl)-1-(R)-1-méthyléthyl]-2-(R)-2-hydroxy-2-(3-chlorophényl)-éthanamine ou un sel de celle-ci acceptable du point de vue pharmaceutique, mélangée à une quantité aussi faible que possible de l'isomère SS, et éventuellement en mélange avec l'isomère RS ou l'isomère SR, où l'isomère RS forme jusqu'à 50% en poids du mélange des isomères RR et RS ou bien l'isomère SR forme jusqu'à 50% en poids du mélange des isomères RR et SR, caractérisé en ce que le procédé comprend :
(A) la réaction d'un composé de formule (IA) avec un composé de formule (IB) : 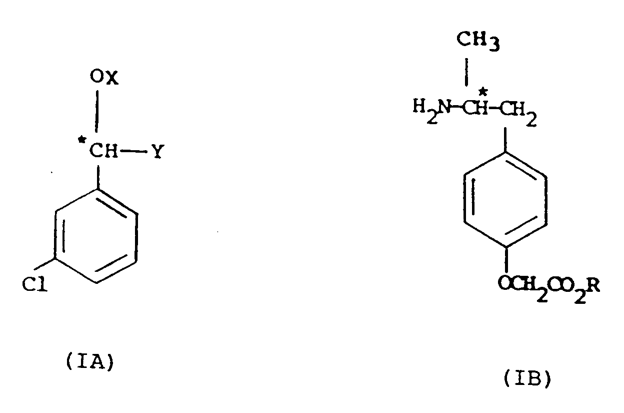 dans lesquelles X est un atome d'hydrogène et Y est un groupe -CH₂R^{x} dans lequel R^{x} est un groupe mobile, ou X représente avec Y une partie >CH₂ et R est un atome d'hydrogène ou un groupe protecteur, et soit l'un des atomes de carbone marqués d'un astérisque a la configuration R et l'autre atome de carbone marqué d'un astérisque est un atome de carbone racémique, soit les deux atomes de carbone marqués d'un astérisque ont la configuration R; ou
(B) la réduction d'un composé de formule (II) : 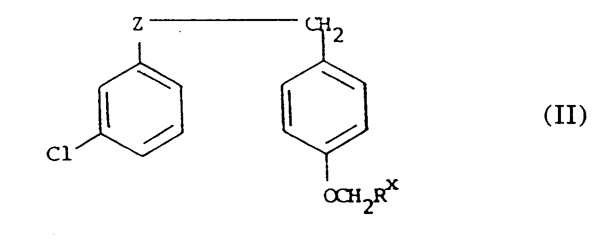 dans laquelle R^{x} est un groupe carboxy ou une forme protégée de celui-ci et Z représente une partie de formule (c), (d), (e) ou (f) : 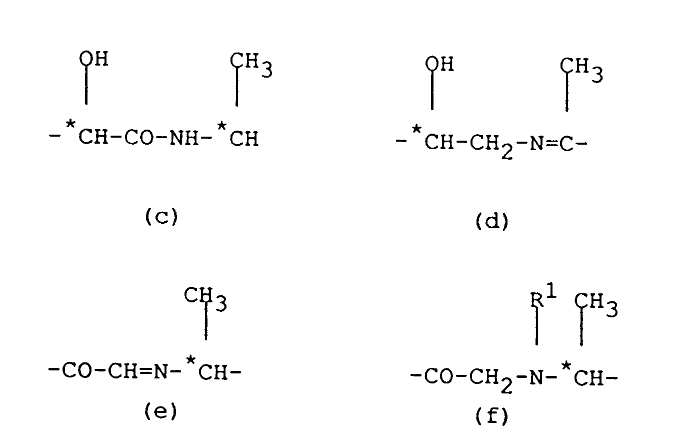 et dans les composés de formule (II) dans laquelle Z est un partie (c), soit l'un des atomes de carbone marqués d'un astérisque a la configuration R et l'autre atome de carbone marqué d'un astérisque est un atome de carbone racémique, soit les deux atomes de carbone marqués d'un astérisque ont la configuration R; et dans les composés dans lesquels Z est une partie (d), (e) ou (f), l'atome de carbone marqué d'un astérisque a la configuration R; R¹ dans la partie (f) représente un atome d'hydrogène ou un groupe protecteur;
et ensuite, si cela est requis, la réalisation d'une ou de plusieurs des étapes facultatives suivantes :
(i) séparation d'un mélange quelconque d'épimères;
(ii) élimination d'un groupe protecteur quelconque;
(iii) formation d'un groupe carboxy à partir d'un quelconque groupe carboxy protégé;
(iv) formation d'un ester méthylique d'un quelconque groupe -OCH₂CO₂H;
(v) formation d'un sel acceptable du point de vue pharmaceutique du produit ainsi formé.

8. L'isomère RR, suivant la revendication 1, éventuellement en mélange avec l'isomère SS suivant la revendication 1, dans lequel l'isomère SS forme moins de 50% en poids du mélange des isomères RR et SS, utilisable en tant que substance thérapeutique active.

9. L'isomère RR, suivant la revendication 1, éventuellement en mélange avec l'isomère SS suivant la revendication 1, dans lequel l'isomère SS forme moins de 50% en poids du mélange des isomères RR et SS, utilisable dans le traitement de l'hyperglycémie chez l'homme ou des mammifères non humains.

10. L'isomère RR, suivant la revendication 1, éventuellement en mélange avec l'isomère SS suivant la revendication 1, dans lequel l'isomère SS forme moins de 50% en poids du mélange des isomères RR et SS, utilisable dans le traitement de l'obésité de l'homme ou des mammifères non humains.

11. Composition pharmaceutique, comprenant l'isomère RR, suivant la revendication 1, éventuellement en mélange avec l'isomère SS suivant la revendication 1, dans lequel l'isomère SS forme moins de 50% en poids du mélange des isomères RR et SS, la composition comprenant aussi un support acceptable du point de vue pharmaceutique.

12. Méthode pour améliorer la prise de poids et/ou améliorer le rendement de l'utilisation de la nourriture et/ou augmenter la masse de viande maigre des animaux sur pied, qui comprend l'administration à ces animaux sur pied, d'une quantité non toxique efficace de l'isomère RR, suivant la revendication 1, éventuellement en mélange avec l'isomère SS suivant la revendication 1, dans lequel l'isomère SS forme moins de 50% en poids du mélange des isomères RR et SS.

13. L'isomère RR, suivant la revendication 1, éventuellement en mélange avec l'isomère SS suivant la revendication 1, dans lequel l'isomère SS forme moins de 50% en poids du mélange des isomères RR et SS, utilisable pour diminuer le taux de mortalité à la naissance et augmenter le taux de survie post-natale des animaux sur pied.

14. Formulation de prémélange acceptable du point de vue vétérinaire, comprenant l'isomère RR, suivant la revendication 1, éventuellement en mélange avec l'isomère SS suivant la revendication 1, dans lequel l'isomère SS forme moins de 50% en poids du mélange des isomères RR et SS, la formulation de prémélange comprenant aussi un support acceptable du point de vue vétérinaire pour celle-ci.

15. L'isomère RR, suivant la revendication 1, éventuellement en mélange avec l'isomère SS suivant la revendication 1, dans lequel l'isomère SS forme moins de 50% en poids du mélange des isomères RR et SS, utilisable dans le traitement de l'hyperglycémie ou de l'obésité chez l'homme ou des mammifères non humains.

## Revendications (Revendications pour l'(les) Etat(s) contractant(s) suivant(s): ES)

1. Procédé pour préparer la N-[2-(4-carboxyméthoxyphényl)-1-(R)-1-méthyléthyl]-2-(R)-2-hydroxy-2-(3-chlorophényl)-éthanamine ou un sel de celle-ci acceptable du point de vue pharmaceutique, ("l'isomère RR"), éventuellement en mélange avec la N-[2-(4-carboxyméthoxyphényl)-1-(S)-1-méthyléthyl]-2-(s)-2-hydroxy-2-(3-chlorophényl)-éthanamine ou un sel de celle-ci acceptable du point de vue pharmaceutique, ("l'isomère SS"), où l'isomère SS forme moins de 50% en poids du mélange des isomères RR et SS, lequel procédé comprend :
la résolution d'un mélange racémique de l'isomère RR et de l'isomère SS; ou
procédé pour préparer la N-[2-(4-carboxyméthoxyphényl)-1-(R)-1-méthyléthyl]-2-(R)-2-hydroxy-2-(3-chlorophényl)-éthanamine ou un sel de celle-ci acceptable du point de vue pharmaceutique, mélangée à une quantité aussi faible que possible de l'isomère SS, et éventuellement en mélange avec l'isomère RS ou l'isomère SR, où l'isomère RS forme jusqu'à 50% en poids du mélange des isomères RR et RS ou bien l'isomère SR forme jusqu'à 50% en poids du mélange des isomères RR et SR, lequel procédé comprend :
(A) la réaction d'un composé de formule (IA) avec un composé de formule (IB) : 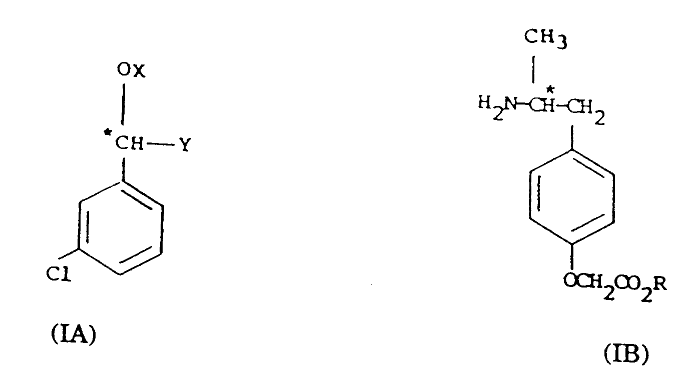 dans lesquelles X est un atome d'hydrogène et Y est un groupe -CH₂R^{x} dans lequel R^{x} est un groupe mobile, ou X représente avec Y une partie >CH₂ et R est un atome d'hydrogène ou un groupe protecteur, et soit l'un des atomes de carbone marqués d'un astérisque a la configuration R et l'autre atome de carbone marqué d'un astérisque est un atome de carbone racémique, soit les deux atomes de carbone marqués d'un astérisque ont la configuration R; ou
(B) la réduction d'un composé de formule (II) : 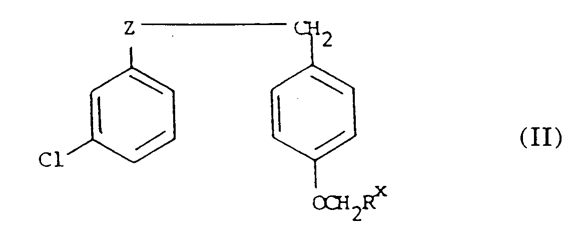 dans laquelle R^{x} est un groupe carboxy ou une forme protégée de celui-ci et Z représente une partie de formule (c), (d), (e) ou (f) : 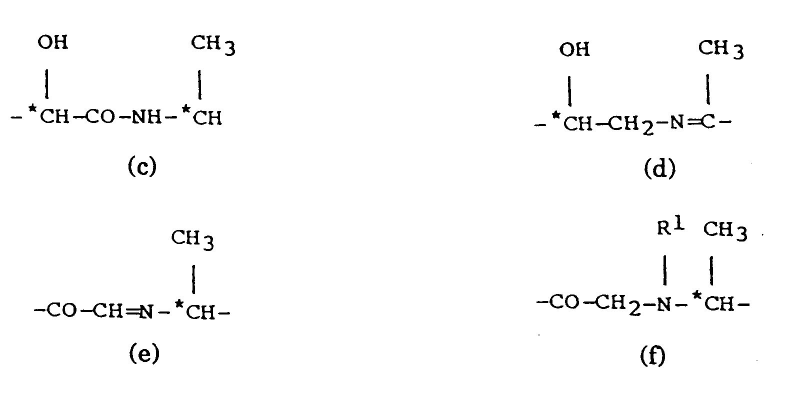 et dans les composés de formule (II) dans laquelle Z est un partie (c), soit l'un des atomes de carbone marqués d'un astérisque a la configuration R et l'autre atome de carbone marqué d'un astérisque est un atome de carbone racémique, soit les deux atomes de carbone marqués d'un astérisque ont la configuration R; et dans les composés dans lesquels Z est une partie (d), (e) ou (f), l'atome de carbone marqué d'un astérisque a la configuration R; R¹ dans la partie (f) représente un atome d'hydrogène ou un groupe protecteur;
et ensuite, si cela est requis, la réalisation d'une ou de plusieurs des étapes facultatives suivantes :
(i) séparation d'un mélange quelconque d'épimères;
(ii) élimination d'un groupe protecteur quelconque;
(iii) formation d'un groupe carboxy à partir d'un quelconque groupe carboxy protégé;
(iv) formation d'un sel acceptable du point de vue pharmaceutique du produit ainsi formé.

2. Procédé suivant la revendication 1, dans lequel l'isomère SS forme 0 à 45%, 0 à 40% ou 0 à 30% en poids du mélange.

3. Procédé suivant les revendications 1 ou 2, dans lequel l'isomère SS forme 0 à 10% en poids du mélange.

4. Procédé suivant l'une quelconque des revendications 1 à 3, dans lequel l'isomère SS forme 0 à 2% en poids du mélange.

5. Procédé suivant l'une quelconque des revendications 1 à 4, dans lequel l'isomère RR est mélangé à une quantité aussi faible que possible de l'isomère SS.

6. Procédé suivant l'une quelconque des revendications 1 à 5, dans lequel l'isomère RR est sous une forme optiquement pure.

7. L'isomère RR, suivant la revendication 1, éventuellement en mélange avec l'isomère SS suivant la revendication 1, dans lequel l'isomère SS forme moins de 50% en poids du mélange des isomères RR et SS, utilisable en tant que substance thérapeutique active.

8. L'isomère RR, suivant la revendication 1, éventuellement en mélange avec l'isomère SS suivant la revendication 1, dans lequel l'isomère SS forme moins de 50% en poids du mélange des isomères RR et SS, utilisable dans le traitement de l'hyperglycémie chez l'homme ou des mammifères non humains.

9. L'isomère RR, suivant la revendication 1, éventuellement en mélange avec l'isomère SS suivant la revendication 1, dans lequel l'isomère SS forme moins de 50% en poids du mélange des isomères RR et SS, utilisable dans le traitement de l'obésité de l'homme ou des mammifères non humains.

10. Méthode pour améliorer la prise de poids et/ou améliorer le rendement de l'utilisation de la nourriture et/ou augmenter la masse de viande maigre et/ou diminuer le taux de mortalité à la naissance et augmenter le taux de survie post-natale des animaux sur pied, qui comprend l'administration à ces animaux sur pied, d'une quantité non toxique efficace de l'isomère RR, suivant la revendication 1, éventuellement en mélange avec l'isomère SS suivant la revendication 1, dans lequel l'isomère SS forme moins de 50% en poids du mélange des isomères RR et SS.

11. Méthode pour diminuer le taux de mortalité à la naissance et augmenter le taux de survie post-natale des animaux sur pied, qui comprend l'administration à des animaux gravides, d'une quantité non toxique efficace de l'isomère RR, suivant la revendication 1, éventuellement en mélange avec l'isomère SS suivant la revendication 1, dans lequel l'isomère SS forme moins de 50% en poids du mélange des isomères RR et SS.
